# EUROPÄISCHE PATENTSCHRIFT

(11) **EP 3 917 356 B1**
(45) Veröffentlichungstag und Bekanntmachung des Hinweises auf die Patenterteilung: **25.09.2024**
(21) Anmeldenummer: 20702774.9
(22) Anmeldetag: 29.01.2020
(51) Int. Cl.: A45D 29/05, A61B 17/54, A61C 1/10, A61C 1/14, A61B 90/00

(54) **VAKUUM-HANDSTÜCK FÜR EIN MEDIZINISCHES ODER KOSMETISCHES BEARBEITUNGSGERÄT**
HANDPIECE FOR A MEDICAL OR COSMETIC PROCESSING DEVICE
POIGNÉE POUR UN APPAREIL DE TRAITEMENT MÉDICAL OU COSMÉTIQUE

(30) Priorität: 29.01.2019 DE 102019102179
(43) Veröffentlichungstag der Anmeldung: 08.12.2021
(73) Patentinhaber: NWT Management GmbH, 04425 Taucha (DE)
(72) Erfinder: LAMOUREUX, Bruno, 1180 Bruxelles (BE); BAUCH, Dieter, 76751 Jockgrim (DE); PAUFLER, André, 04249 Leipzig (DE)
(74) Vertreter: Maikowski & Ninnemann Patentanwälte Partnerschaft mbB
(86) Internationale Anmeldenummer: PCT/EP2020/052155
(87) Internationale Veröffentlichungsnummer: WO 2020/157125

(56) Entgegenhaltungen:
- EP-A1- 0 670 149
- EP-A1- 1 362 559
- EP-A2- 0 920 839
- DE-A1- 102006 023 187
- DE-A1- 19 652 190
- DE-A1- 3 311 193
- DE-A1- 3 402 585
- DE-U1- 202014 007 414

## Beschreibung

### Gebiet der Erfindung

Die Erfindung betrifft ein Vakuum-Handstück für ein medizinisches oder kosmetisches Bearbeitungsgerät, aufweisend eine Anschlussleitung an einem Anschlussende, ein Gehäuse, in dem zwischen einem Anschlussende und einem Bearbeitungsende ein Aufnahmeraum vorgesehen ist, in dem eine Rotationsantriebseinheit zum Antrieb eines am Bearbeitungsende anbringbaren Rotationswerkzeuges aufnehmbar ist. Das Handstück ist so gestaltet, dass es von dem Anschlussende trennbar ist, wobei insbesondere der Motor als Teil der Rotationsantriebseinheit an dem Anschlussende verbleibt und der abnehmbare Teil des Handstücks separat sterilisierbar, insbesondere hitze- oder dampfsterilisierbar ist. Das Handstück weist ferner eine neuartige Drehspanneinrichtung auf, in der das Rotationswerkzeug anbringbar ist.

### Hintergrund der Erfindung

In der Körperpflege werden Einrichtungen zur Beseitigung von z.B. Hornhaut an Füßen oder Händen verwendet, mit denen diese Hornhaut abgefräst oder abgeschliffen wird. Die Werkzeuge werden mit hoher Drehzahl betrieben. Geräte mit einem ähnlichen Grundsätzlichen Aufbau finden im medizinischen, wie beispielsweise dem zahnärztlichen Bereich Anwendung. Um eine gute Handhabung des Werkzeuges zu erhalten, sind die bekannten Geräte derart aufgebaut, dass ein Geräteteil einen Transformator und eine Drehzahlregelung sowie die elektronischen Bauelemente und einen Behälter, z.B. Beutel oder ähnliches, zur Aufnahme des gefrästen oder abgeschliffenen Staubes aufnimmt und dass dieser Geräteteil über einen Leitungsstrang mit einem Handstück verbunden ist. In diesem Handstück ist ein elektrischer Motor, in der Regel ein Niederspannungsmotor angeordnet, über den der Antrieb des Werkzeuges erfolgt.

Die bekannten Einrichtungen haben verschiedene Nachteile. Infolge einer starren Verbindung oder einer festen Verankerung des Werkzeuges, z.B. über eine druckfederbelastete Einspannung, zwischen Spanneinheit und Motoreinheit ist das Handstück nicht von der Motoreinheit trennbar. Bei anderen bekannten Einrichtungen erfolgt die Einspannung des Werkzeuges umständlich durch ein Festdrehen eines Spannelementes unter Verwendung eines separaten Werkzeugs. Bei bekannten Einrichtungen erfolgt die Absaugung über in dem Handstück angeordnete Kanäle und ist damit durch die Dimensionierung der Kanäle festgelegt. Die bekannten Einrichtungen haben den Nachteil, dass sie infolge ihres Aufbaues nur mit verhältnismäßig hohem Aufwand repariert werden können und infolge der Anordnung der Staubabsaugkanäle ein im Durchmesser relativ großes Handstück verlangen und damit unhandlich werden. Ein weiterer besonderer Nachteil als Folge der oben genannten nicht trennbaren Verbindung zwischen Motor und Spanneinheit ist, dass die Handstücke nicht in einem befriedigenden Maß gereinigt und hitze- oder dampfsterilisiert werden können und somit die zur Behandlung verschiedener Personen mit dem gleichen Handstück erforderlichen hygienischen Anforderungen nicht erfüllt werden können.

Die DE 3311193 A1 beschreibt eine Einrichtung zur Fußpflege, bei der das Handstück zwar von der Einrichtung trennbar ist. Die Motoreinheit befindet sich nach dem Abtrennen jedoch fest verbaut in dem Handstück, wodurch dieses so gestaltete Handstück nicht hitze- oder dampfsterilisierbar ist.

Die EP 2664288 B1 offenbart ein Motorhandstück eines Bearbeitungsgerätes für die humanmedizinische oder kosmetische Fußpflege, die Chirurgie oder für den Dentalbereich. Dieses Motorhandstück weist einen Aufnahmeraum auf, in dem ein elektrischer Motor sowie eine Lagerung zum Antrieb eines am Bearbeitungsende anbringbaren Werkzeuges enthalten sind. Der Teil des Handstücks, der von dem Rest der Einrichtung separierbar ist, enthält wie in der EP 2664288 B1 beschrieben einen fest verbauten Motor und ist somit nicht hitze- oder dampfsterilisierbar.

Auch die DE 202004005966 U1 offenbart ein Handstück für ein medizinisches oder kosmetisches Bearbeitungsgerät, dass eine Rotationsantriebseinheit innerhalb eines Gehäuses aufweist, wobei dieses Gehäuse beispielsweise mittels eines Bajonettverschlusses lösbar mit dem Rest des Gehäusekörpers verbunden ist. Auch bei dem hier beschriebenen Handstück verbleibt die Rotationsantriebseinheit einschließlich Motor in dem Teil des Handstücks, der von dem Rest der Einrichtung abnehmbar ist. Somit ist auch das in der DE 202004005966 U1 offenbarte Handstück nicht hitze- oder dampfsterilisierbar.

### Beschreibung der Erfindung

Die Aufgabe der Erfindung bestand nunmehr darin, die Nachteile des Standes der Technik zu überwinden und insbesondere ein Handstück für ein medizinisches oder kosmetisches Bearbeitungsgerät bereitzustellen, das einfach zu reinigen und hitze- oder dampfsterilisierbar ist und das ein einfaches, komfortables Auswechseln von Rotationswerkzeugen ohne zusätzliches Werkzeug ermöglicht.

Diese Aufgabe wird gelöst durch ein Vakuum-Handstück für ein medizinisches oder kosmetisches Bearbeitungsgerät gemäß Anspruch 1.

Die Ausführungsform als Spray-Handstück fällt nicht unter den in den Ansprüchen beanspruchten Gegenstand der Erfindung. Es wird nur als Referenz in der Beschreibung erwähnt, fällt jedoch nicht unter den in Anspruch 1 genannten Bereich.

Das erfindungsgemäße Vakuum-Handstück weist eine Anschlussleitung an einem Anschlussende, ein Gehäuse, in dem zwischen einem Anschlussende und einem Bearbeitungsende ein Aufnahmeraum vorgesehen ist, in dem eine Rotationsantriebseinheit zum Antrieb eines am Bearbeitungsende anbringbaren Rotationswerkzeuges aufnehmbar ist, auf. Das Handstück ist so gestaltet, dass es von dem Anschlussende trennbar ist, wobei insbesondere der Motor als Teil der Rotationsantriebseinheit an dem Anschlussende verbleibt und der abnehmbare Teil des Handstücks separat sterilisierbar, insbesondere hitze- oder dampfsterilisierbar ist. Das Handstück weist ferner eine neuartige Drehspanneinrichtung auf, in der das Rotationswerkzeug anbringbar ist.

Das erfindungsgemäße Handstück ist auf den Gebieten der Pediküre, Fußpflege, Zahntechnik, im Dentallabor oder jedem anderen Bereich verwendbar, in dem der Abrieb eines Materials erforderlich ist. Die Rotation eines Rotationswerkzeugs, wie eines Fräsers, Schleifers oder Bohrers erfordert einen rotierenden mechanischen Antrieb, der durch einen Elektromotor oder Luft erzeugt wird. Das Rotationswerkzeug wird durch ein mechanisches Spannsystem im Handstück gehalten. Vorzugsweise enthält das erfindungsgemäße Handstück zum Antrieb des Rotationswerkzeuges einen Mikromotor, der elektrisch betrieben wird. Das Handstück ist vom Anschlussende, und damit von dem am Anschlussende befindlichen Mikromotor abnehmbar. In einer bevorzugten Ausführungsform ist auch der Mikromotor separat von dem Anschlussende und damit von der Anschlussleitung abnehmbar. Während des Gebrauchs verursacht der durch das Rotationswerkzeug erzeugte Abrieb in einigen Fällen Staub und/oder Hitze. In der Praxis werden Einrichtungen eingesetzt, die entweder Vorrichtungen enthalten, um den entstehenden Staub durch Absaugen während des Gebrauchs des Handstücks zu entfernen, oder die Vorrichtungen enthalten, die eine Spray- oder Sprühvorrichtung aufweisen, mit der der anfallende Staub während des Gebrauchs mittels einer Flüssigkeit gebunden wird und gleichzeitig eine Kühlung der Behandlungsstelle erfolgen kann. Die beiden Ausführungsformen der in der Praxis angewendeten Einrichtungen enthalten oftmals auch eine Beleuchtungseinrichtung, um die Sichtbarkeit der Bearbeitungsstelle für den Benutzer während des Gebrauchs des Handstücks zu gewährleisten. Das erfindungsgemäße Handstück weist immer eine neuartige Drehspanneinrichtung auf, die Gegenstand der vorliegenden Erfindung ist. Die Drehspanneinrichtung ist sowohl in Handstücke einbaubar, die einen Mikromotor mit Absaugung aufweisen, als auch in Handstücke, die einen Mikromotor mit Spray- oder Sprühvorrichtung und eine Beleuchtungseinrichtung aufweisen.

Die Rotationsantriebseinheit des erfindungsgemäßen Handstücks weist einen Motor, vorzugsweise einen Elektromotor, insbesondere bevorzugt einen elektrisch betriebenen Mikromotor auf. Ein weiterer Bestandteil der Rotationsantriebseinheit ist eine Lagerhülse, in der die Drehspanneinrichtung aufnehmbar ist. Die Drehspanneinrichtung verfügt in Richtung des Anschlussendes über eine Kupplung, die lösbar mit der Motorwelle verbunden ist und die in Richtung des Bearbeitungsendes eine Spannzange aufweist, in der das Rotationswerkzeug anbringbar ist. In einer bevorzugten Ausführungsform handelt es sich bei der Kupplung an dem Anschlussende der Drehspanneinrichtung um eine Klauenkupplung, die in ein entsprechend geformtes Gegenstück an der Motorwelle des Motors der Rotationsantriebseinheit eingreift und somit eine Drehmomentübertragung mittels Formschluss ermöglicht.

Ein weiterer Aspekt der Erfindung betrifft eine Drehspanneinrichtung, die es ermöglicht, auf einfache Art und Weise ein Rotationswerkzeug in dem erfindungsgemäßen Handstück auszuwechseln. Dazu weist die Drehspanneinrichtung Vorrichtungen, wie beispielsweise eine Spindelhülse auf, in der die Spannzange in Richtung des Bearbeitungsendes des Handstücks aufnehmbar ist und eine Kupplung zu der Motorwelle des Motors der Rotationsantriebseinheit in Richtung des Anschlussendes des Handstücks enthält. Die Spindelhülse ist vorzugsweise in zwei Kugellagern gelagert, die an der Drehspanneinrichtung in Richtung Anschlussende des Handstücks und in Richtung Bearbeitungsende vorgesehen sind. Spannzange und Kupplung sind jeweils mittels Klemmhülse innerhalb eines Langloches axial verschiebbar mit der Spindelhülse verbunden bzw. verbindbar. Die Spannzange ist axial mittels einer Spannfeder gegen die Spindelhülse vorgespannt. Die Kupplung ist axial federnd gelagert und gegen die Spindelhülse in Richtung Anschlussende mittels Druckfeder vorgespannt.

Die erfindungsgemäße Drehspanneinrichtung ermöglicht es auf einfache Art und Weise, ein Rotationswerkzeug einzuspannen oder zum Auswechseln freizugeben. Die Drehspanneinrichtung weist dafür in Richtung des Anschlussendes entsprechend geeignete Mittel auf, die es ermöglichen, eine Drehbewegung des Gehäuses des Handstücks auf die Drehspanneinrichtung im Inneren des Handstücks zu übertragen und dort in eine axiale Bewegung der Spannzange umzuwandeln. In einer Ausführungsform der Erfindung wird dies durch eine Ausrückeinheit realisiert, die Bestandteil der Drehspanneinrichtung ist. Diese Ausrückeinheit besteht in einer besonders bevorzugten Ausführungsform aus zwei Teilen, einer Schiebehülse und einer Drehhülse. Schiebe- und Drehhülse weisen jeweils sich gegenüberliegende deckungsgleiche Wendelflächen (Helikoide) mit gleicher Steigung auf. Durch Drehen der Drehhülse werden Drehhülse und Schiebehülse gegeneinander verdreht und dadurch eine axiale Verschiebung der Spannzange innerhalb der Drehspanneinrichtung erzeugt. Dadurch kommt es zum Spannen oder Entspannen der Spannzange und somit zum Befestigen oder Freigeben eines Rotationswerkzeuges in der Spannzange. Ein geeignetes Mittel zur Übertragung der Drehbewegung von dem Gehäuse auf die Ausrückeinheit der Drehspanneinrichtung ist vorzugsweise ein Mitnehmerstift, der in eine dafür vorgesehene Öffnung der Drehhülse eingebracht werden kann. Bei diesem Mitnehmerstift kann es sich zum Beispiel um einen Metallstift oder ein Metallbolzen handeln, der in eine dafür vorgesehene Öffnung der Drehhülse eingeschraubt ist. Die Drehspanneinrichtung weist vorzugsweise weitere Mittel auf, die ein Mitdrehen der Spannzange beim Drehen der Ausrückeinheit verhindern. Die erfindungsgemäße Drehspanneinrichtung hat den Vorteil, dass ein sehr einfaches, aber optimales Festklemmen eines Rotationswerkzeuges vorgenommen werden kann. Die Drehspanneinrichtung können so ausgeführt sein, dass eine Drehung des Klemmaktuators um bis zu 270° erfolgt. Es hat sich jedoch herausgestellt, dass es ausreichend ist, wenn der Klemmaktuator eine Drehung um bis zu 180°, vorzugsweise um bis zu 90° vollführt. Mit einer Drehung um 90° wird ein optimales Festklemmen eines Rotationswerkzeuges in der Spannzange gewährleistet.

Die erfindungsgemäße Drehspanneinrichtung ist in einer besonders bevorzugten Ausführungsform als eigenständige Einheit ausgeführt, wodurch die gesamte Drehspanneinrichtung des erfindungsgemäßen Handstücks herausnehmbar und damit auswechselbar ist. Um dies zu gewährleisten, kann die Drehspanneinrichtung in Richtung des Anschlussendes des Handstücks weitere Dichtungen, Sicherungselemente und Befestigungselemente aufweisen.

In einer bevorzugten Ausführungsform der Erfindung ist die Drehspanneinrichtung in einer Lagerhülse im Inneren des Gehäuses des Handstücks angeordnet, wobei die Drehspanneinrichtung in Richtung des Bearbeitungsendes und in Richtung des Anschlussendes des Handstücks mittels Sicherungselementen, Befestigungselementen und Lagern gesichert, befestigt und gelagert ist. Die Festlegung des Ausmaßes bzw. des Drehwinkels der Drehbewegung der Drehhülse der Drehspanneinrichtung wird vorteilhafterweise auf sehr einfache Weise gelöst. Die Lagerhülse, in der die Drehspanneinrichtung angeordnet ist, weist ein Langloch zur Führung des Mitnehmerstiftes auf, der mit der Drehhülse der Drehspanneinrichtung im Inneren der Lagerhülse verbunden ist. Die Anordnung des Mitnehmerstiftes und die Dimensionierung des Langlochs legen das Ausmaß der Drehbewegung der Drehhülse der Drehspanneinrichtung fest. Vorzugsweise ist die Drehhülse, wie oben beschrieben, um 90° drehbar.

Wie bereits beschrieben, besteht ein besonderer Vorteil des erfindungsgemäßen Handstücks darin, dass das Handstück ohne Motor von dem Anschlussende entkoppelt werden kann, damit eine bessere Reinigung und Sterilisation, insbesondere Hitzesterilisation erfolgen kann. Vorzugsweise weist die Lagerhülse deshalb an dem zum Anschlussende des Handstücks hin gerichteten Ende Mittel zum An- bzw. Entkuppeln des Handstücks vom Anschlussende auf. Dabei kann es sich um übliche Bajonetteinrichtungen, Schnappverschlüsse oder eine Schraubverbindung handeln. Das Anschlussende weist vorzugsweise entsprechend passende Gegenstücke auf.

Weiterhin beschrieben wird ein Spray-Handstück. In diesem Fall weist das Handstück Einrichtungen für die Durchleitung von Wasser und Druckluft, und optional von Licht auf. Die Durchleitung von Wasser und Druckluft kann über Kanäle, Röhren oder Schläuche erfolgen. Die Durchleitung von Licht erfolgt vorzugsweise über Lichtleiter. Diese Mittel zur Durchleitung von Wasser, Druckluft und Licht münden am Bearbeitungsende des Handstücks in einer Nase, die Mittel zur Abdichtung, sowie eine Luftdüse und eine Wasserdüse aufweist. Der Lichtleiter mündet ebenfalls in dieser Nase. Die Mittel zur Durchleitung von Wasser, Druckluft und Licht werden im Inneren des Gehäuses des Handstücks außen an der Lagerhülse vorbei geführt. Das Anschlussende, das mit der Anschlussleitung verbunden ist, enthält entsprechende Vorrichtungen, mit denen die Mittel zur Durchleitung von Wasser, Licht und Druckluft verbunden werden können, so dass die Durchleitung von Wasser, Licht und Druckluft von der Anschlussleitung bis hin zu der Nase und den Düsen, d.h. vom Anschlussende bis zum Bearbeitungsende des Handstücks gewährleistet ist. Ein solches Mittel kann beispielsweise ein Multikupplungselement sein, in das einzelne Kupplungen zur Verbindung von Versorgungsleitungen für Wasser, Druckluft, Strom und Licht mit den dafür vorgesehenen Mitteln zur Durchleitung der jeweiligen Medien durch das Handstück gebündelt enthalten sind. Es kann auch vorgesehen sein, dass Wasser und Druckluft gemeinsam als Sprüh-Fluid in einer Leitung, Kanal, Röhre oder Schlauch durch das Handstück geleitet werden und am Bearbeitungsende des Handstücks in einer einzigen Sprühdüse münden. Mit der durch das Handstück hindurchgeleiteten Sprühflüssigkeit wird einerseits eine Kühlwirkung des Arbeitsbereichs erzielt. Gleichzeitig wird aber auch eine Kühlung des Motors in dem Handstück erreicht.

Wenn ein Handstück als Spray-Handstück ausgebildet ist, so weist das Gehäuse vorzugsweise mehrere Teile, besonders bevorzugt drei Teile auf, wobei der mittlere Teil als drehbare Hülse ausgebildet ist. Der Mitnehmerstift, der im Inneren an der Drehhülse der Ausrückeinheit angeordnet ist, greift in eine dafür vorgesehene Vertiefung im Inneren des Spannrings des Gehäuses ein. Durch Drehen des Spannrings erfolgt gleichzeitig die Drehung der Drehhülse der Ausrückeinheit in der Drehspanneinrichtung im Inneren des erfindungsgemäßen Handstücks, wodurch die Spannzange spannbar oder lösbar ist und ein Rotationswerkzeug eingespannt oder freigegeben werden kann. Besonders bevorzugt ist es, wenn die mittlere drehbare Hülse Griffmulden aufweist. Dadurch wird die Handhabung des erfindungsgemäßen Handstücks verbessert und es kann vermieden werden, dass der Benutzer beim Spannen und Entspannen der Spannzange, d.h. beim Drehen der mittleren Hülse aufgrund von vorhandener Feuchtigkeit oder von vorhandenem Staub abrutscht.

Gemäß der Erfindung ist das Handstück als Vakuum-Handstück ausgebildet.

Am Anschlussende ist ein Vakuumschlauchverbinder zum Anschließen eines Absaugschlauches angeordnet.

Bei dem erfindungsgemäßen Vakuum-Handstück ist grundsätzlich eine Drehspanneinrichtung vorgesehen, die identisch oder ähnlich zu der Drehspanneinrichtung des Spray- Handstücks ist. Das Vakuum- Handstück weist im Inneren ebenfalls Mittel zur Übertragung der Drehbewegung des Gehäuses auf die Drehspanneinrichtung auf. In der Ausführungsform als Vakuum-Handstück ist das Gehäuse vorzugsweise einteilig als äußere Hülse gestaltet. Beim Drehen der äußeren Hülse wird der Mitnehmerstift, der an der Drehhülse der Ausrückeinheit der Drehspanneinrichtung angeordnet ist und der in dafür vorgesehenen Einrichtungen des Gehäuses eingreift, mitgenommen und eine Drehbewegung der Drehhülse erzeugt. Das Vakuum-Handstück kann im Inneren weitere Mittel und Einrichtungen aufweisen, die an der Übertragung der Drehbewegung vom Gehäuse auf die Drehspanneinrichtung mitwirken. Es können weitere Elemente vorgesehen sein, mit denen die äußere Hülse an der Drehspanneinrichtung oder weiteren Bauteilen im Inneren des Gehäuses verankerbar ist. Durch die Drehbewegung der äußeren Hülse ist die Spannzange spannbar oder lösbar, wodurch ein Rotationswerkzeug eingespannt oder freigegeben werden kann. Die Befestigung des Gehäuses, d.h. der äußeren Hülse an dem Anschlussende erfolgt beim Vakuum-Handstück mit herkömmlichen Mitteln, wie beispielsweise einer Bajonettverschluss-Einrichtung, einer lösbaren Rastverbindung, einer Schraubverbindung oder ähnlichem. Zum Entfernen von Schleifstaub und Materialabrieb während des Betriebs des Vakuum-Handstücks ist im Inneren des Handstücks ein Vakuumkanal vorgesehen, der alle Abschnitte des Handstücks sowie den Motorabschnitt bis hin zu dem Vakuumschlauch durchquert, um eine Absaugung über diesen Durchgangskanal zu erreichen. Mit dem Vakuumschlauch ist üblicherweise eine Absaugungsvorrichtung verbunden.

Dem Spray-Handstück und dem erfindungsgemäßen Vakuum-Handstück ist gemein, dass der Motor der Rotationsantriebseinheit mit dem Anschlussende verbunden bleibt, und das Gehäuse des Handstücks ohne den Motor vom Anschlussende abgekoppelt werden. Dazu weist das Handstück in einer weiteren Ausführungsform einen Gehäuseabschnitt für die oben beschriebene Kopplungseinrichtung auf, mit der das Handstück von dem Anschlussende gelöst oder mit dem Anschlussende verbunden werden kann.

Ein weiterer Vorteil des erfindungsgemäßen Handstücks besteht darin, dass der Motor so gestaltet ist, dass er als separates Bauteil von dem Anschlussende auf einfache Art und Weise abnehmbar ist. Damit ist es möglich, das Anschlussende optimal zu reinigen und gegebenenfalls zu sterilisieren und die Motoreinheit zu reparieren oder auszutauschen. Einrichtungen für die Ausführung einer lösbaren Befestigung des Motors an das Anschlussende sind dem Fachmann bekannt.

Die Materialien, aus denen die einzelnen Bauteile des erfindungsgemäßen Handstücks und der erfindungsgemäßen Drehspanneinrichtung bestehen, sind vorzugsweise sterilisierbar, insbesondere hitze- oder dampfsterilisierbar. Für die Konstruktion des erfindungsgemäßen Handstücks kommen somit geeignete Metalle, wie Edelstahl, sowie geeignete hitzeresistente Kunststoffe, wie beispielsweise Polyetheretherketon (PEEK) infrage.

Die Erfindung wird nachfolgend anhand von 8 Figuren näher erläutert. Es zeigen:
- Figur 1: die erfindungsgemäße Drehspanneinrichtung;
- Figur 2: verschiedene Ausführungsformen des erfindungsgemäßen Handstücks für ein medizinisches oder kosmetisches Bearbeitungsgerät,
- Figur 3: Details des Aufbaus eines Spray-Handstücks,
- Figur 4: Details des Aufbaus eines Spray-Handstücks,
- Figur 5: Details des Aufbaus eines erfindungsgemäßen Vakuum-Handstücks,
- Figur 6: die Grundmodule eines Spray- Handstücks,
- Figur 7: die Grundmodule eines erfindungsgemäßen Vakuum-Handstücks, und

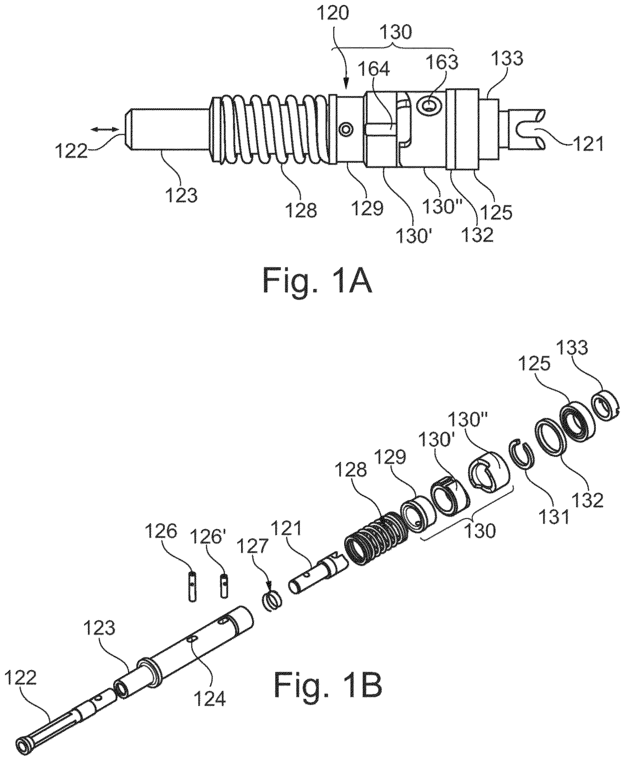

Figur **1A** zeigt eine Übersichtsdarstellung der erfindungsgemäßen Drehspanneinrichtung 120 und Figur **1B** zeigt die Drehspanneinrichtung 120 in Explosionsdarstellung. Die Drehspanneinrichtung 120 weist eine Spindelhülse 123 auf, die eine Spannzange 122 in Richtung des Bearbeitungsendes 103 und eine Kupplung 121 in Richtung des Anschlussendes des Handstücks 100 enthält. Die Kupplung 121 ist in der Spindelhülse 123 gelagert. Spannzange 122 und Kupplung 121 sind mittels Befestigungsmitteln 126, 126' mit der Spindelhülse 123 verbunden und über eine Druckfeder 127 und eine Spannfeder 128 federnd gelagert. Als Befestigungsmittel 126, 126' wurden in der hier gezeigten Ausführungsform Klemmhülsen aus Edelstahl benutzt. Die Drehspanneinrichtung 120 weist weiterhin eine Ausrückeinheit 130 für das Spannen und Entspannen der Spannzange 122 auf. Die Ausrückeinheit 130 besteht aus zwei Teilen einer Schiebehülse 130' und einer Drehhülse 130". Die erfindungsgemäße Drehspanneinrichtung ermöglicht es auf einfache Art und Weise, ein Rotationswerkzeug einzuspannen oder zum Auswechseln freizugeben. Es ist gut zu erkennen, dass die Schiebehülse 130' und die Drehhülse 130" jeweils gegenüberliegende deckungsgleiche Wendelflächen (Helikoide) mit gleicher Steigung aufweisen. Durch Drehen der Drehhülse 130" werden die Drehhülse 130" und die Schiebehülse 130' gegeneinander verdreht und entsprechend der Steigung der Wendelflächen die Schiebehülse 130' axial verschoben. Die Spannzange 122 im Inneren der Spindelhülse 123 ist mittels einer Klemmhülse 126 mit der Spannhülse 129 verbunden, die außen an der Spindelhülse 123 angeordnet ist. Die Spindelhülse 123 weist zu diesem Zweck ein Langloch 124 in axialer Richtung auf, in dem die Klemmhülse 126 geführt ist. Die Spannhülse 129 dient zur Aufnahme der Spannfeder 128 und zur Übertragung der durch die Ausrückeinheit 130 ausgelösten axialen Verschiebung auf die Spannzange 122.

Bei einer Drehbewegung der Drehhülse 130" kommt es zu einer der Federkraft der Spannfeder 128 entgegen wirkenden axialen Verschiebung der Spannhülse 129 und der Spannzange 122 in der Spindelhülse 123 (angedeutet durch den Doppelpfeil an der Spannzange 122). Werden durch die Drehbewegung-der Drehhülse 130"die Wendelflächen der Schiebehülse 130' und der Drehhülse 130" aus der Position der größten Überdeckung heraus zueinander verdreht, nimmt die Überdeckung und damit die Kontaktfläche der Wendelflächen ab und die Spannhülse 129 und Spannzange 122 wird in der Spindelhülse 123 in Richtung des Bearbeitungsendes 103 des erfindungsgemäßen Handstücks 100 geschoben. Dadurch kommt es zum Entspannen der Spannzange 122 und zum Freigeben eines Rotationswerkzeuges (nicht gezeigt) in der Spannzange 122. Das Rotationswerkzeug kann jetzt herausgenommen und ausgetauscht werden. Erfolgt die Drehbewegung der Drehhülse 130" in entgegengesetzter Richtung, d.h. werden die Wendelflächen der Schiebehülse 130' und der Drehhülse 130" wieder übereinander geschoben in Richtung der größten Überdeckung, wird während dieses Vorgangs der Spannring 129 und die Spannzange 122 in der Spindelhülse 123 in Richtung des Anschlussendes 101 des erfindungsgemäßen Handstücks 100 bewegt. Dadurch schließt sich die Spannzange 122 und ein Rotationswerkzeug kann festgeklemmt und arretiert werden. Um die Drehbewegung der Drehhülse 130" zu gewährleisten, weist dieser vorzugsweise einen Mitnehmerstift 146 auf, der in eine dafür vorgesehene Öffnung 163 der Drehhülse 130" eingebracht werden kann. Bei diesem Mitnehmerstift 146 kann es sich zum Beispiel um einen Metallstift oder ein Metallbolzen handeln, der in eine dafür vorgesehene Öffnung 163 der Drehhülse 130" eingeschraubt ist. Um die geradlinige Bewegung der Schiebehülse 130'zu gewährleisten, weist die Schiebehülse 130' vorzugsweise eine Nut 164 auf, in dem bei der geradlinigen axialen Bewegung der Spannzange 122 ein Führungsstift 165 geführt wird. Durch diese Gestaltung der Schiebehülse 130' wird sichergestellt, dass sich diese nicht mitdreht, während eine Drehbewegung an der Drehhülse 130" ausgeführt wird. Während des Betriebes wird eine Berührung der feststehenden Schiebehülse 130' in der Ausrückeinheit 130 mit dem Spannring 129 in der rotierenden Drehspanneinrichtung 120 mit Hilfe der Rückholfeder 144 verhindert, welche sich innen gegen die Lagerhülse 140 abstützt und somit die Schiebehülse 130' gegen die Drehhülse 130" spannt. Während des Betriebes befindet sich die Ausrückeinheit in der Grundstellung, d.h. die sich berührenden Wendelflächen der Drehhülse 130" und der Schiebehülse 130' überdecken sich vollständig. Die Dimensionierung des Langloches 124 in der Spindelhülse 123 bewirkt eine Begrenzung des Weges des Spannringes 129, sodass in der beschriebenen Stellung ein ausreichender Spalt zwischen der feststehenden Schiebehülse 130' und dem rotierenden Spannring 129 sichergestellt ist.

Die Drehspanneinrichtung 120 weist weiterhin in Richtung des Anschlussendes 101 des Handstücks 100 einen SEEGER Sicherungsring 131, einen Ausgleichsring 132 und eine Schlitzmutter 133 zur Befestigung des Kugellagers in der Spindelhülse 123 auf. Es ist gut zu erkennen, dass die Drehspanneinrichtung 120 in Richtung des Anschlussendes 101 des Handstücks 100 eine Klauenkupplung 121 aufweist, welche mittels einer Klemmhülse 126' und einer Druckfeder 127 axial beweglich mit der Spindelhülse 123 verbunden ist. Diese ist dazu geeignet, lösbar mit einem entsprechenden Gegenstück der Motorwelle 111 des elektrischen Antriebsmotors 110 des Handstücks 100 in Wechselwirkung zu treten.

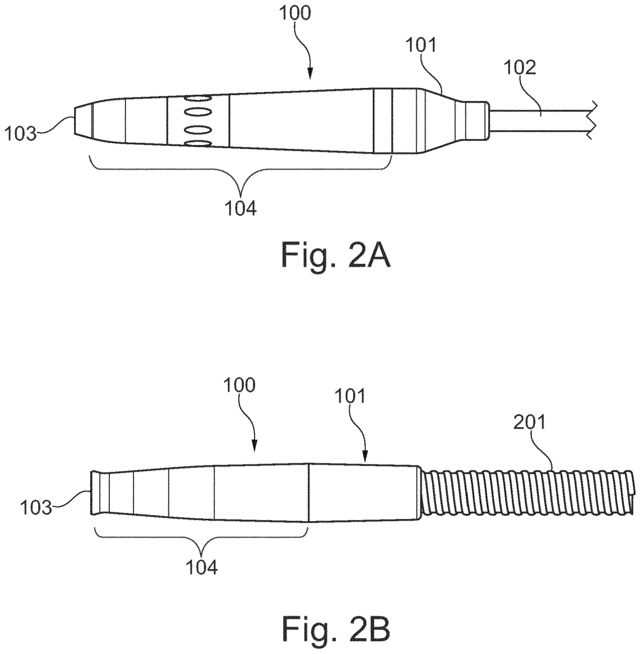

Figur **2A** zeigt ein Handstück 100 eines Spray-Handstücks. Das Spray-Handstück weist ein Gehäuse 104, ein Anschlussende 101 und ein Bearbeitungsende 103 auf. Am Anschlussende befindet sich die Anschlussleitung 102, die das Spray-Handstück mit Strom versorgt und in den Versorgungsleitungen für Wasser und Druckluft für ein Sprüh-Fluid enthalten sind. Das Anschlussende 101 kann weiterhin mit Licht emittierenden Bauteilen ausgestattet sein, wodurch mit Hilfe von Lichtleitern 156 am Bearbeitungsende 103 eine Beleuchtungsfunktion realisiert werden kann.

Figur **2B** zeigt das erfindungsgemäße Vakuum-Handstück 100. Das Vakuum-Handstück weist ein Gehäuse 104, ein Anschlussende 101 und ein Bearbeitungsende 103 auf. An dem Anschlussende 104 ist ein Saugschlauch bzw. Vakuumschlauch angebracht, der mit einer entsprechenden Saugpumpe (nicht gezeigt) verbunden ist. Das Vakuum-Handstück ist so aufgebaut, dass während des Betriebs Schleifstäube und Abrieb, die am Bearbeitungsende entstehen, durch das gesamte Handstück 100 hindurch in den Vakuumschlauch 201 gesaugt werden.

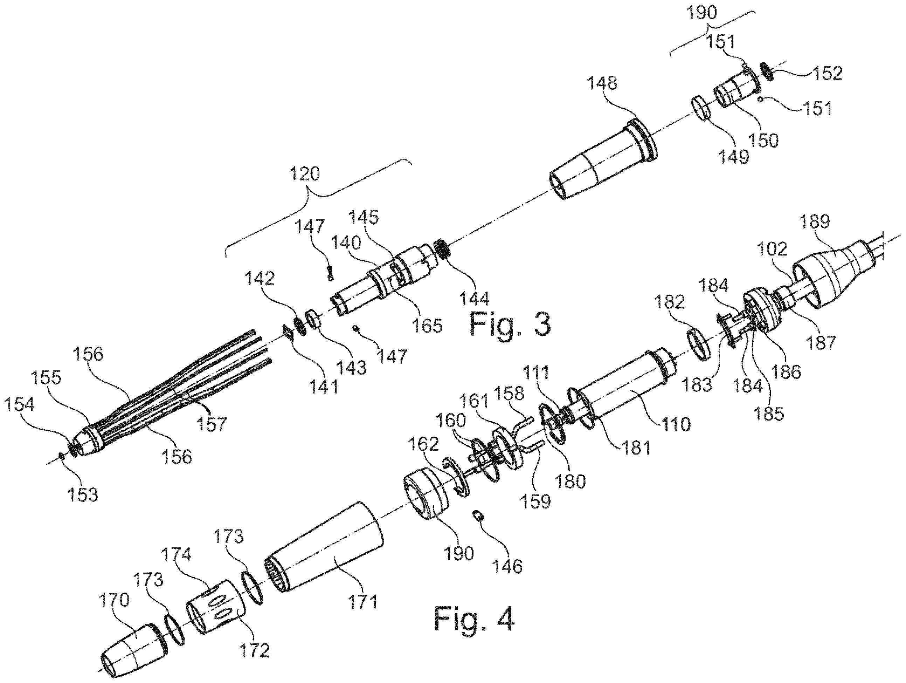

Figur **3** zeigt weitere Details des Aufbaus des Handstücks 100 als Spray-Handstück. Die Drehspanneinrichtung 120 ist in einer Lagerhülse 140 des Gehäuses 104 des Handstücks 100 gelagert, wobei die Drehspanneinrichtung 120 in Richtung des Bearbeitungsendes 103 und in Richtung des Anschlussendes 101 des Handstücks 100 jeweils mittels Kugellager 125 und 143, einer Wellenfederscheibe 141, einem Ausgleichsring 142 und Gewindestiften 147 gesichert und gelagert ist. Die Lagerhülse 140 weist ein Langloch 145 zur Aufnahme eines Mitnehmerstiftes 146 auf, der mit der Drehspanneinrichtung 120 im Inneren der Lagerhülse 140 verbunden ist. Durch die Anordnung des Mitnehmerstiftes 146 in dem Langloch 145 und durch die Dimensionierung des Langlochs 145 wird die Richtung und das Ausmaß der Drehbewegung der Drehspanneinrichtung 120 festgelegt.

Die Lagerhülse 140 weist in Richtung des Anschlussendes 101 eine Motorführungshülse 148 sowie Mittel zum An- bzw. Entkuppeln des Handstücks vom Anschlussende, hier ausgeführt als Federhülse 149, Rastkörper 150 mit Kugeln 151 und eine Dichtung, hier ausgeführt als O-Ring 152, auf.

Das als Spray-Handstück ausgebildete Handstück 100 weist weiterhin Einrichtungen für die Durchleitung von Wasser und Druckluft, und optional Licht auf. Die Durchleitung von Wasser und Druckluft erfolgt über dafür vorgesehene Kanäle (Wasserkanal 158 und Luftkanal 159). Die Kanäle für Wasser 158 und Luft 159 sind in der hier gezeigten Ausführungsform mit einem Kupplungsrings 161 lösbar verbunden. Die Durchleitung von Licht erfolgt vorzugsweise über Lichtleiter, wie beispielsweise einen Glasfaserstab 156. Die Kanäle 158 und 159 zur Durchleitung von Wasser, Druckluft und die Lichtleiter 156 münden am Bearbeitungsende 103 des Handstücks 100 in eine Nase 155, die Dichtungen 153, 154 sowie eine Luftdüse 105' und eine Wasserdüse 106' aufweist. Die Lichtleiter 156 münden ebenfalls in dieser Nase 155. Die Mittel zur Durchleitung von Wasser, und Druckluft sind im Inneren des Gehäuses 104 des Handstücks 100 als Kapillarröhrchen 157 ausgebildet und werden außen an der Lagerhülse 140 vorbeigeführt. Eine Verbindung zu den Kanälen 158 und 159 werden über Rohverbinder 160, die sich an dem Kupplungsring 161 befinden, hergestellt. Gleiches trifft für die Lichtleiter 156 zu. Das Anschlussende 101, das mit der Anschlussleitung 102 verbunden ist, enthält entsprechende Vorrichtungen, mit denen die Mittel zur Durchleitung von Wasser (157, 158), Licht (156) und Druckluft (157, 159) verbunden werden können, so dass die Durchleitung von Wasser, Licht und Druckluft von der Anschlussleitung 102 bis hin zu der Nase 155 und den Düsen 105`, 106`, d.h. vom Anschlussende 101 bis zum Bearbeitungsende 103 des Handstücks 100 gewährleistet ist. Ein solches Mittel kann beispielsweise ein Multikupplungselement sein, in das einzelne Kupplungen zur Verbindung von Versorgungsleitungen für Wasser, Druckluft, Strom und Licht mit den dafür vorgesehenen Mitteln zur Durchleitung der jeweiligen Medien durch das Handstück gebündelt enthalten sind. In der hier gezeigten Ausführungsform enthält das Handstück jedoch ein gesondertes Bauteil für die Erzeugung von Licht, nämlich ein PCB-Board 162 mit LEDs. Das Licht der LEDs wird mittels der Glasfaserstäbe 156 von dem PCB-Board 162 zum Bearbeitungsende 103 des Handstücks 100 geleitet.

Figur 4 zeigt weitere Details des Aufbaus des Handstücks 100 als Spray-Handstück. Das Gehäuse 104 weist in Richtung des Bearbeitungsendes 103 eine vordere Hülse 170 und in Richtung des Anschlussendes 101 eine hintere Hülse 171 auf. Zwischen der vorderen Hülse 170 und der hinteren Hülse 171 ist eine mittlere Hülse 172 drehbar angeordnet. Zwischen vorderer Hülse 170 und mittlerer Hülse 172 und zwischen hinterer Hülse 171 und mittlerer Hülse 172 ist jeweils O-Ring 173 als Dichtungselement angeordnet. Der Mitnehmerstift 146 greift in eine dafür vorgesehene Vertiefung im Inneren der mittleren Hülse 172 ein. Durch Drehen der mittleren Hülse 172 ist die Spannzange 122 spannbar oder lösbar. Dadurch kann ein Rotationswerkzeug 210 eingespannt oder freigegeben werden. Die mittlere Hülse 172 weist in der hier gezeigten Ausführungsform Griffmulden 174 auf. Dadurch wird die Handhabung des Handstücks 100 verbessert.

Figur **4** zeigt weiterhin den Motor 110 der Rotationsantriebseinheit mit Motorwelle 111. Es ist gut zu erkennen, dass die Motorwelle 111 ein entsprechend geformtes Gegenstück zur Klauenkupplung 121 der Drehspanneinrichtung 120 bildet. Der Motor wird mit einem Haltering 180, einem O-Ring 181, einer Abstandshülse 182 in dem Handstück 100 befestigt. Das Anschlussende 101 weist weitere Befestigungsmittel wie Innensechskantschrauben 184 auf, sowie eine Steckverbindung 185 und isolierte Buchsen 186. Zur Befestigung der Anschlussleitung 102 wird eine Schlauchsicherungsbuchse 187 verwendet. Das Anschlussende 101 wird durch eine Schraubkappe 189 geschützt.

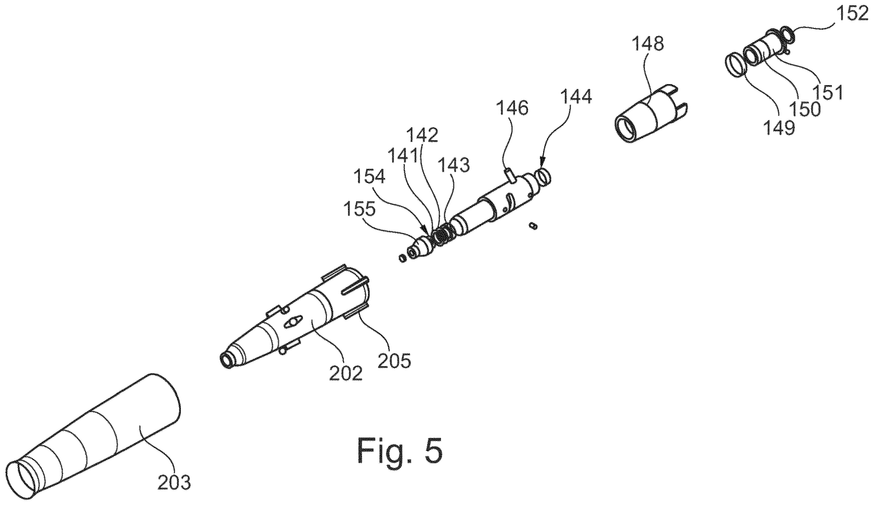

Figur **5** zeigt weitere Details des Aufbaus des erfindungsgemäßen Vakuum-Handstücks 100. Am Anschlussende 101 ist ein Vakuumschlauchverbinder 200 zum Anschließen eines Absaugschlauches 201 vorgesehen. Das Vakuum-Handstück weist eine innere Hülse 202 auf, in der die Lagerhülse 140 mit der Drehspanneinrichtung 120 untergebracht ist. Der Mitnehmerstift 146 der Drehspanneinrichtung 120 greift in eine dafür vorgesehene Vertiefung im Inneren der inneren Hülse 202 ein. Das Vakuum-Handstück weist eine äußere Hülse 203 auf, die im Inneren Elemente enthält, die mit Rastelementen 204 und Mitnehmern 205 auf der Außenseite der inneren Hülse 202 wechselwirken, wodurch die äußere Hülse 203 auf der inneren Hülse 202 verankerbar ist. Durch Drehen der äußeren Hülse 203 ist die Spannzange 122 spannbar oder lösbar. Dadurch kann ein Rotationswerkzeug 210 eingespannt oder freigegeben werden.

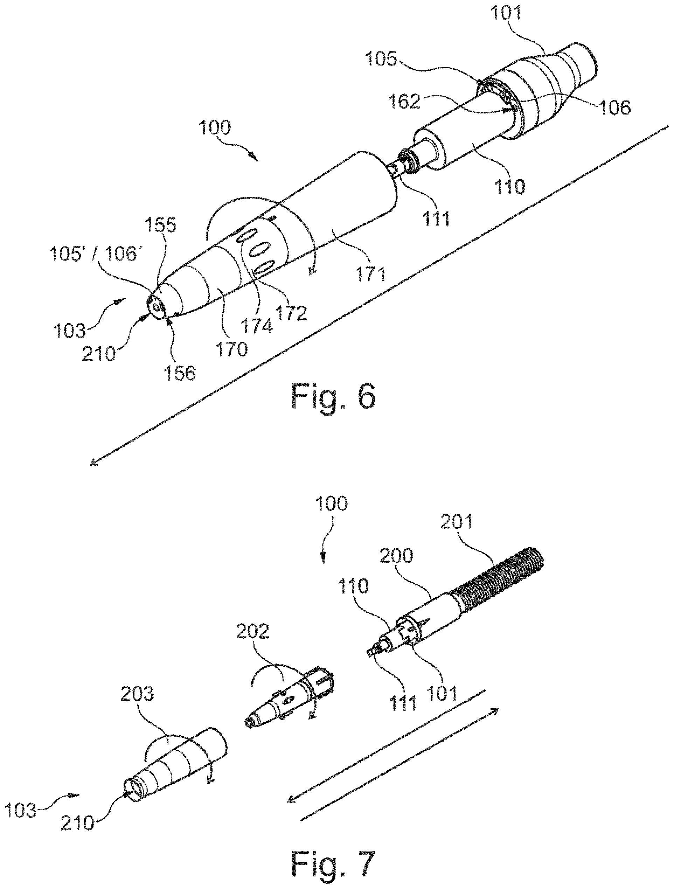

Figur 6 zeigt eine weitere Ansicht des Aufbaus des Spray-Handstücks 100 mit Anschlussende 101. Der Motor 110 mit Motorwelle 111 verbleibt nach Entkuppeln des Gehäuses des Handstücks 100 am Anschlussende 101. Am Anschlussende 101 befinden sich ein Luftanschluss 105, ein Wasseranschluss 106 und das PCB-Board mit LEDs 162. Zu erkennen ist das dreiteilige Gehäuse, umfassend eine vordere Hülse 170, eine mittlere drehbare Hülse 172 und eine hintere Hülse 171. Die mittlere drehbare Hülse ist zur besseren Handhabung, insbesondere beim Einspannen und Ausspannen eines Rotationswerkzeuges, mit Griffmulden 174 versehen. In Richtung des Bearbeitungsendes 103 befindet sich die Nase 155, die Sprühdüsen für Luft 105'und für Wasser 106' sowie einen Austritt für die Lichtleiter 156 aufweisen. Am Bearbeitungsende 103 kann sich ein Rotationswerkzeug 210 befinden. Der vom Anschlussende 101 zum Bearbeitungsende 103 verlaufende Pfeil deutet an, dass Wasser und Luft für die Sprüheinrichtung sowie Licht durch das gesamte Handstück 100 hindurch geleitet werden. Da der Motor 110 bei Ablösen des Handstücks 100 an dem Anschlussende 101 verbleibt, kann der ablösbare Teil des Handstücks 100 in vorteilhafter Weise gereinigt und sterilisiert, insbesondere hitze- oder dampfsterilisiert werden.

Figur 7 zeigt eine weitere Ansicht des Aufbaus des erfindungsgemäßen Vakuum-Handstücks 100 mit Vakuumschlauch 201, der mit einem Vakuumschlauchverbinder 200 am Anschlussende 101 des Handstücks 100 angeschlossen ist. Auch in diesem Fall verbleibt der Motor 110 am Anschlussende 101, wenn der vordere Teil des Handstücks 100, hier gebildet durch die äußere Hülse 203 und die innere Hülse 202 vom Anschlussende 101 abgelöst werden. Die innere Hülse 202 enthält die erfindungsgemäße Drehspanneinrichtung 120. Der durchgehende Pfeil vom Bearbeitungsende 103 in Richtung Anschlussende 101 deutet den Verlauf der Luft sowie von Schleifstäuben und Abrieb an, die während des Gebrauchs des Handstücks 100 vom Bearbeitungsende 103 durch das gesamte Handstück hindurch in den Vakuumschlauch 201 hineingesaugt werden. Da der Motor 110 bei Ablösen des Handstücks 100 an dem Anschlussende 101 verbleibt, können die ablösbaren Teile 203, 202 des Handstücks 100 in vorteilhafter Weise gereinigt und sterilisiert, insbesondere hitze- oder dampfsterilisiert werden. Das durch die Erfindung bereitgestellte Handstück weist mehrere Vorteile auf. Die Drehspanneinrichtung 120 stellt sicher, dass ein Rotationswerkzeug optimal eingespannt aber auch für den Anwender auf einfache Art und Weise vom Handstück 100 gelöst werden kann. Dadurch, dass das Handstück 100 derart vom Anschlussende 101 gelöst werden kann, dass der Motor 110 Anschlussende verbleibt, wird sichergestellt, dass der Teil des Handstücks 100, an dem Verunreinigungen durch den Gebrauch des Handstücks 100 im kosmetischen oder medizinischen Bereich auftreten, optimal gereinigt und sterilisiert, insbesondere hitze- oder dampfsterilisiert werden kann.

### Liste der Bezugszeichen

- 100: Handstück
- 101: Anschlussende
- 102: Anschlussleitung
- 103: Bearbeitungsende
- 104: Gehäuse
- 105: Luftanschluss
- 106: Wasseranschluss
- 105': Luftdüse
- 106': Wasserdüse
- 110: Motor
- 111: Motorwelle
- 120: Drehspanneinrichtung
- 121: Kupplung, Klauenkupplung
- 122: Spannzange
- 123: Spindelhülse
- 124: Langloch für Spannzange
- 125: Kugellager
- 126,126`: Klemmhülse, Befestigungsmittel
- 127: Druckfeder
- 128: Spannfeder
- 129: Spannhülse
- 130: Ausrückeinheit
- 130': Schiebehülse
- 130": Drehhülse
- 131: SEEGER Sicherungsring
- 132: Ausgleichsring
- 133: Schlitzmutter
- 140: Lagerhülse
- 141: Wellenfederscheibe
- 142: Ausgleichsring
- 143: Kugellager
- 144: Rückholfeder
- 145: Langloch
- 146: Mitnehmerstift
- 147: Gewindestift
- 148: Motorführungshülse
- 149: Federhülse
- 150: Rastkörper
- 151: Kugeln
- 152: Dichtung, O-Ring
- 153: Dichtung
- 154: Dichtung, O-Ring
- 155: Nase
- 156: Glasfaserstab, Lichtleiter
- 157: Kapillarröhrchen
- 158: Wasserkanal
- 159: Luftkanal
- 160: Rohrverbinder, Schlauchverbinder
- 161: Kupplungsring, Verbindungsring
- 162: PCB-Board mit LEDs
- 163: Bohrung, Öffnung
- 164: Nut
- 165: Führungsstift
- 170: vordere Hülse
- 171: hintere Hülse
- 172: mittlere drehbare Hülse
- 173: Dichtung, O-Ring
- 174: Griffmulde
- 180: Haltering
- 181: Dichtung, O-Ring
- 182: Abstandshülse
- 183: PCB Kontaktierung
- 184: Innensechskantschraube
- 185: Steckverbindung
- 186: isolierende Buchse
- 187: Schlauchsicherungsbuchse
- 189: Schraubkappe
- 190: Gehäuseabschnitt für Kupplungseinrichtung zwischen Gehäuse des Handstücks und Anschlussende
- 200: Vakuumschlauchverbinder
- 201: Vakuumschlauch
- 202: innere Hülse
- 203: äußere Hülse
- 204: Rastelemente
- 205: Mitnehmer
- 210: Rotationswerkzeug, Aufnahme für das Rotationswerkzeug

## Patentansprüche

1. Vakuum-Handstück (100) für ein medizinisches oder kosmetisches Bearbeitungsgerät, aufweisend:
- eine Anschlussleitung (102) an einem Anschlussende (101),
- ein Gehäuse (104), in dem zwischen einem Anschlussende (101) und einem Bearbeitungsende (103) ein Aufnahmeraum vorgesehen ist, in dem eine Rotationsantriebseinheit zum Antrieb eines am Bearbeitungsende (103) anbringbaren Rotationswerkzeuges (210) aufnehmbar ist,
wobei das Handstück (100) am Anschlussende (101) einen Vakuumschlauchverbinder (200) zum Anschließen eines Absaugschlauches (201) aufweist;
wobei das Handstück (100) von dem Anschlussende (101) trennbar ist, wobei das Handstück (100) so gestaltet ist, dass ein Motor (110) als Teil der Rotationsantriebseinheit nach Abkopplung des Gehäuses (104) an dem Anschlussende (101) verleibt;
wobei das Handstück eine Drehspanneinrichtung (120) aufweist, in der das Rotationswerkzeug (210) anbringbar ist,
wobei das Handstück (100) eine innere Hülse (202) aufweist, in der eine Lagerhülse (140) mit der Drehspanneinrichtung (120) untergebracht ist, und der Mitnehmerstift (146) in eine dafür vorgesehene Vertiefung im Inneren der inneren Hülse (202) eingreift,
wobei das Handstück (100) eine äußere Hülse (203) aufweist, die im Inneren Elemente enthält, die mit Rastelementen (204) und Mitnehmern (205) auf der Außenseite der inneren Hülse (202) wechselwirken, wodurch die äußere Hülse (203) auf der inneren Hülse (202) verankerbar ist und wobei durch Drehen der äußeren Hülse (203) eine Spannzange (122) spannbar oder lösbar ist und das Rotationswerkzeug (210) eingespannt oder freigegeben werden kann.

2. Vakuum-Handstück (100) nach Anspruch 1,
wobei die Rotationsantriebseinheit einen Motor (110), vorzugsweise einen Elektromotor, und eine Lagerhülse (140) aufweist, in der die Drehspanneinrichtung (120) aufnehmbar ist, die in Richtung des Anschlussendes (101) über eine Kupplung (121), vorzugsweise eine Klauenkupplung, lösbar mit der Motorwelle (111) verbunden ist und die in Richtung des Bearbeitungsendes (103) die Spannzange (122) aufweist, in der das Rotationswerkzeug (210) anbringbar ist.

3. Vakuum-Handstück (100) nach Anspruch 1 oder 2, wobei die Drehspanneinrichtung (120) eine Spindelhülse (123), die die Spannzange (122) in Richtung des Bearbeitungsendes (103) des Handstücks (100) enthält, und eine Kupplung (121) in Richtung des Anschlussendes (101) des Handstücks (100) enthält, aufweist, die Spindelhülse (123) in zwei Lagern (125) und (145) gelagert ist, wobei Spannzange (122) und Kupplung (121) mittels Befestigungsmitteln (126, 126') mit der Spindelhülse (123) verbunden sind und über eine Druckfeder (127) und eine Spannfeder (128) federnd gelagert sind.

4. Vakuum-Handstück (100) nach einem der vorherigen Ansprüche, wobei die Drehspanneinrichtung (120) weiterhin eine Spannhülse (129) mit einer Ausrückeinheit (130) für das Spannen und Entspannen der Spannzange (122) aufweist und die Drehspanneinrichtung (120) in Richtung des Anschlussendes (101) des Handstücks (100) Dichtungen und Sicherungselemente (131, 132, 133) zur Sicherung von Spannzange (122), Kupplung (121) und Ausrückeinheit (130) in der Spindelhülse (123) aufweist.

5. Vakuum-Handstück (100) nach einem der vorherigen Ansprüche, wobei die Drehhülse (130") der Drehspanneinrichtung (120) in der Lagerhülse (140) des Gehäuses (104) des Handstücks (100) drehbar gelagert ist, wobei die Drehspanneinrichtung (120) in Richtung des Bearbeitungsendes (103) und in Richtung des Anschlussendes (101) des Handstücks (100) mittels Sicherungselementen (141, 142, 147), Lagern (125, 143) und Federn (144) gesichert und gelagert ist.

6. Vakuum-Handstück (100) nach Anspruch 5, wobei die Lagerhülse (140) ein Langloch (145) zur Aufnahme des Mitnehmerstiftes (146) aufweist, der mit der Drehhülse (130") der Drehspanneinrichtung (120) im Inneren der Lagerhülse (140) verbunden ist, wobei durch die Anordnung des Mitnehmerstiftes (146) in dem Langloch (145) und durch die Dimensionierung des Langlochs (145) die Richtung und das Ausmaß der Drehbewegung der Drehhülse (130") der Drehspanneinrichtung (120) festlegbar ist.

7. Vakuum-Handstück (100) nach Anspruch 6, wobei Schiebehülse (130') und Drehhülse (130") so gestaltet sind, dass die an der Drehhülse (130") erzeugbare Drehbewegung in eine axiale Bewegung der Spannzange (122) umwandelbar ist und dadurch die Spannzange (122) spannbar oder lösbar ist.

8. Vakuum-Handstück (100) nach einem der vorherigen Ansprüche, wobei die Lagerhülse (140) in Richtung des Anschlussendes (101) eine Motorführungshülse (148) sowie Mittel (149, 150, 151, 152) zum An- bzw. Entkuppeln des Handstücks (100) vom Anschlussende (101) aufweist.

9. Vakuum-Handstück (100) nach einem der vorherigen Ansprüche, wobei der Motor (110) mit dem Anschlussende (101) verbunden ist und das Gehäuse (104) des Handstücks (100) weiterhin einen Gehäuseabschnitt (190) für eine Kupplungseinrichtung aufweist, mit der das Handstück (100) von dem Anschlussende (101) gelöst oder mit dem Anschlussende (101) verbunden werden kann.

10. Vakuum-Handstück (100) nach einem der vorherigen Ansprüche, wobei die Bauteile des abnehmbaren Teils des Handstücks (100) aus Materialien bestehen, die hitze-oder dampfsterilisierbar sind, wie beispielsweise Edelstahl oder hitzetolerierenden Kunststoffen, wie zum Beispiel PEEK.

## Claims

1. Vacuum handpiece (100) for a medical or cosmetic processing device, having:
- a connection line (102) at a connecting end (101);
- a housing (104) in which a receptacle space is provided between a connecting end (101) and a processing end (103), a rotary drive unit for driving a rotary tool (210) that is attachable to the processing end (103) being able to be received in said receptacle space;
wherein the handpiece (100) at the connecting end (101) has a vacuum hose connector (200) for connecting a suction hose (201); wherein the handpiece (100) is able to be separated from the connecting end (101), wherein the handpiece (100) is designed such that a motor (110), as part of the rotary drive unit, upon decoupling of the housing (104) remains on the connecting end (101);
wherein the handpiece has a rotary clamping installation (120) in which the rotary tool (210) is able to be attached;
wherein the handpiece (100) has an inner sleeve (202) in which a bearing sleeve (140) with the rotary clamping installation (120) is accommodated, and the entrainment pin (146) engages in a depression which is provided to this end in the interior of the inner sleeve (202);
wherein the handpiece (100) has an outer sleeve (203) which in the interior contains elements which interact with latching elements (204) and entrainment elements (205) on the external side of the inner sleeve (202), as a result of which the outer sleeve (203) is able to be anchored on the inner sleeve (202), and wherein a collet chuck (122) is able to be tensioned or released, and the rotary tool (210) can be clamped or released, by rotating the outer sleeve (203).

2. Vacuum handpiece (100) according to Claim 1,
wherein the rotary drive unit has a motor (110), preferably an electric motor, and a bearing sleeve (140) in which the rotary clamping installation (120) is able to be received and which in the direction of the connecting end (101) by way of a coupling (121), preferably a dog clutch, is releasably connected to the motor shaft (111) and which in the direction of the processing end (103) has the collet chuck (122) in which the rotary tool (210) is able to be attached.

3. Vacuum handpiece (100) according to Claim 1 or 2,
wherein the rotary clamping installation (120) has a spindle sleeve (123) which in the direction of the processing end (103) of the handpiece (100) contains the collet chuck (122), and in the direction of the connecting end (101) of the handpiece (100) contains a coupling (121); the spindle sleeve (123) is mounted in two bearings (125) and (145);
wherein the collet chuck (122) and the coupling (121) are connected to the spindle sleeve (123) by means of fastening means (126, 126') and are resiliently mounted by way of a compression spring (127) and a tension spring (128).

4. Vacuum handpiece (100) according to one of the preceding claims,
wherein the rotary clamping installation (120) furthermore has a tensioning sleeve (129) with a release unit (130) for clamping and relaxing the collet chuck (122), and the rotary clamping installation (120) in the direction of the connecting end (101) of the handpiece (100) has seals and locking elements (131, 132, 133) for locking the collet chuck (122), the coupling (121) and the release unit (130) in the spindle sleeve (123).

5. Vacuum handpiece (100) according to one of the preceding claims,
wherein the rotary sleeve (130") of the rotary clamping installation (120) is rotatably mounted in the bearing sleeve (140) of the housing (104) of the handpiece (100), wherein the rotary clamping installation (120) in the direction of the processing end (103) and in the direction of the connecting end (101) of the handpiece (100) is locked and mounted by means of locking elements (141, 142, 147), bearings (125, 143) and springs (144) .

6. Vacuum handpiece (100) according to Claim 5,
wherein the bearing sleeve (140) has an elongate bore (145) for receiving the entrainment pin (146) which is connected to the rotary sleeve (130") of the rotary clamping installation (120) in the interior of the bearing sleeve (140), wherein the direction and the extent of the rotating movement of the rotary sleeve (130") of the rotary clamping installation (120) is able to be established by the disposal of the entrainment pin (146) in the elongate bore (145) and by the dimensions of the elongate bore (145).

7. Vacuum handpiece (100) according to Claim 6,
wherein the sliding sleeve (130') and the rotary sleeve (130") are designed such that the rotating movement that is able to be generated on the rotary sleeve (130") is able to be converted into an axial movement of the collet chuck (122) and, as a result thereof, the collet chuck (122) is able to be tensioned or released.

8. Vacuum handpiece (100) according to one of the preceding claims,
wherein the bearing sleeve (140) in the direction of the connecting end (101) has a motor-guiding sleeve (148) as well as means (149, 150, 151, 152) for coupling or decoupling, respectively, the handpiece (100) to or from the connecting end (101) .

9. Vacuum handpiece (100) according to one of the preceding claims,
wherein the motor (110) is connected to the connecting end (101), and the housing (104) of the handpiece (100) furthermore has a housing portion (190) for a coupling installation by way of which the handpiece (100) can be released from the connecting end (101) or connected to the connecting end (101).

10. Vacuum handpiece (100) according to one of the preceding claims,
wherein the components of the detachable part of the handpiece (100) are composed of materials which are able to be sterilized by heat or steam, such as, for example, stainless steel or heat-tolerant plastics materials such as, for example, PEEK.

## Revendications

1. Pièce à main à vide (100) pour un appareil de traitement médical ou cosmétique, présentant :
- une ligne de raccordement (102) à une extrémité de raccordement (101) ;
- un boîtier (104) dans lequel est prévu, entre une extrémité de raccordement (101) et une extrémité de traitement (103), un espace de réception dans lequel peut être reçue une unité d'entraînement en rotation pour entraîner un outil rotatif (210) pouvant être monté sur l'extrémité de traitement (103),
la pièce à main (100) présentant, à l'extrémité de raccordement (101), un connecteur de tuyau à vide (200) pour raccorder un tuyau d'aspiration (201) ;
la pièce à main (100) étant séparable de l'extrémité de raccordement (101), la pièce à main (100) étant conçue de telle sorte qu'un moteur (110), faisant partie de l'unité d'entraînement en rotation, reste à l'extrémité de raccordement (101) après découplage du boîtier (104) ;
la pièce à main présentant un dispositif de serrage rotatif (120) dans lequel l'outil rotatif (210) peut être monté ;
la pièce à main (100) présentant un manchon intérieur (202) dans lequel est logé un manchon de palier (140) avec le dispositif de serrage rotatif (120), et la broche d'entraînement (146) s'engage dans un renfoncement prévu à cet effet à l'intérieur du manchon intérieur (202),
la pièce à main (100) présentant un manchon extérieur (203) qui contient à l'intérieur des éléments qui interagissent avec des éléments d'encliquetage (204) et des entraîneurs (205) sur le côté extérieur du manchon intérieur (202), moyennant quoi le manchon extérieur (203) peut être ancré sur le manchon intérieur (202) et, par rotation du manchon extérieur (203), une pince de serrage (122) pouvant être serrée ou détachée et l'outil rotatif (210) pouvant être serré ou libéré.

2. Pièce à main à vide (100) selon la revendication 1,
l'unité d'entraînement en rotation présentant un moteur (110), de préférence un moteur électrique, et un manchon de palier (140) dans lequel peut être reçu le dispositif de serrage rotatif (120) qui, en direction de l'extrémité de raccordement (101), est relié de manière amovible à l'arbre du moteur (111) par un accouplement (121), de préférence un accouplement à griffes, et qui, en direction de l'extrémité de traitement (103), présente la pince de serrage (122) dans laquelle l'outil rotatif (210) peut être monté.

3. Pièce à main à vide (100) selon la revendication 1 ou 2,
le dispositif de serrage rotatif (120) présentant un manchon de broche (123) contenant la pince de serrage (122) en direction de l'extrémité de traitement (103) de la pièce à main (100) et un accouplement (121) en direction de l'extrémité de raccordement (101) de la pièce à main (100), le manchon de broche (123) étant monté dans deux paliers (125) et (145),
la pince de serrage (122) et l'accouplement (121) étant reliés au manchon de broche (123) au moyen de moyens de fixation (126, 126') et étant montés élastiquement par l'intermédiaire d'un ressort de compression (127) et d'un ressort de tension (128).

4. Pièce à main à vide (100) selon l'une quelconque des revendications précédentes,
le dispositif de serrage rotatif (120) présentant en outre un manchon de serrage (129) avec une unité de débrayage (130) pour le serrage et le desserrage de la pince de serrage (122) et le dispositif de serrage rotatif (120) présentant, en direction de l'extrémité de raccordement (101) de la pièce à main (100), des joints d'étanchéité et des éléments de blocage (131, 132, 133) pour bloquer la pince de serrage (122), l'accouplement (121) et l'unité de débrayage (130) dans le manchon de broche (123).

5. Pièce à main à vide (100) selon l'une quelconque des revendications précédentes,
le manchon rotatif (130") du dispositif de serrage rotatif (120) étant monté de manière rotative dans le manchon de palier (140) du boîtier (104) de la pièce à main (100), le dispositif de serrage rotatif (120) étant bloqué et monté en direction de l'extrémité de traitement (103) et en direction de l'extrémité de raccordement (101) de la pièce à main (100) au moyen d'éléments de blocage (141, 142, 147), de paliers (125, 143) et de ressorts (144).

6. Pièce à main à vide (100) selon la revendication 5,
le manchon de palier (140) présentant un trou oblong (145) pour recevoir la broche d'entraînement (146) qui est reliée au manchon rotatif (130") du dispositif de serrage rotatif (120) à l'intérieur du manchon de palier (140), la direction et l'ampleur du mouvement de rotation du manchon rotatif (130") du dispositif de serrage rotatif (120) pouvant être déterminées par l'agencement de la broche d'entraînement (146) dans le trou oblong (145) et par le dimensionnement du trou oblong (145).

7. Pièce à main à vide (100) selon la revendication 6,
le manchon coulissant (130') et le manchon rotatif (130") étant conçus de telle sorte que le mouvement de rotation pouvant être généré sur le manchon rotatif (130") puisse être converti en un mouvement axial de la pince de serrage (122) et que la pince de serrage (122) puisse ainsi être serrée ou détachée.

8. Pièce à main à vide (100) selon l'une quelconque des revendications précédentes,
le manchon de palier (140) présentant, en direction de l'extrémité de raccordement (101), un manchon de guidage de moteur (148) et des moyens (149, 150, 151, 152) pour coupler et découpler la pièce à main (100) de l'extrémité de raccordement (101) .

9. Pièce à main à vide (100) selon l'une quelconque des revendications précédentes,
le moteur (110) étant relié à l'extrémité de raccordement (101) et le boîtier (104) de la pièce à main (100) présentant en outre une section de boîtier (190) pour un dispositif d'accouplement avec lequel la pièce à main (100) peut être détachée de l'extrémité de raccordement (101) ou reliée à l'extrémité de raccordement (101).

10. Pièce à main à vide (100) selon l'une quelconque des revendications précédentes,
les composants de la partie amovible de la pièce à main (100) étant constitués de matériaux qui peuvent être stérilisés par la chaleur ou la vapeur, tels que par exemple l'acier inoxydable ou des matières plastiques tolérant la chaleur, tels que par exemple le PEEK.
